# DEMANDE DE BREVET EUROPEEN

(11) **EP 2 077 138 A1**
(43) Date de publication de la demande: **08.07.2009**
(21) Numéro de dépôt: 08150007.6
(22) Date de dépôt: 02.01.2008
(51) Int. Cl.: A61Q 3/02, A61K 8/37, A61K 8/73

(54) **Composition cosmétique anhydre**

(71) Demandeur: International Lacquers S.A., 3225 Bettembourg (LU)
(72) Inventeur: Derosier, Frédéric, 57000, Metz (FR); Delas, Christophe, 3926, Mondercange (LU)
(74) Mandataire: Office Freylinger

(57) **Abrégé**

Composition cosmétique anhydre pour vernis à ongles, comprenant au moins un solvant organique cosmétiquement acceptable, de la nitrocellulose et une quantité efficace d'au moins un agent de réduction du jaunissement, dans laquelle au moins un agent de réduction du jaunissement est un dérivé triester de l'acide trimellitique choisi parmi les trimellitates d'alcools gras, procédé de préparation et utilisation de cette composition.

## Description

### Domaine technique

La présente invention concerne une composition cosmétique anhydre et transparente, applicable sur les ongles ou sur un vernis coloré. Cette composition forme, après séchage, une fine couche sur l'ongle ou sur le vernis préalablement appliqué.

### Etat de la technique

Ce type de formulation peut avoir pour objet l'augmentation de la tenue lorsqu'elle est appliquée sur l'ongle ou une augmentation de la brillance et une protection du vernis lorsqu'elle est appliquée sur celui-ci. Ce type de formulation peut également être utilisé seule pour apporter une meilleure brillance à l'ongle.

Une grande majorité de ce type de formules, que l'on appelle généralement « incolores », ou encore « base coat » lorsqu'elle est appliquée avant un vernis à ongles coloré ou encore « top coat » lorsqu'elle est appliquée sur un vernis coloré qui contient des solvants, des résines, un ou plusieurs plastifiants et de la nitrocellulose. Mais l'inconvénient de ce type de formule est la stabilité optique lorsqu'elle est conservée dans les flacons.

La nitrocellulose est un polymère de choix pour la préparation des vernis à ongles puisqu'elle possède des propriétés filmogènes intéressantes, apporte du brillant et se solubilise facilement dans les différents solvants mis en oeuvre. Ce polymère issu d'un produit naturel - la cellulose - a toutefois un inconvénient de taille, puisqu'il entraîne un jaunissement important de la formulation. Ce jaunissement peut être vu comme un défaut de la formulation par le consommateur ou la consommatrice.

La nitrocellulose est préparée par réaction de la cellulose (du bois ou du coton) et de l'acide nitrique en présence d'acide sulfurique dans l'eau. Les étapes de synthèse comprennent également des étapes de filtrage et de lavage pour éliminer les sous-produits de la réaction. La réaction de l'acide nitrique et de la cellulose est en fait une réaction d'estérification des groupes hydroxyles de la cellulose. Considérant la différence de réactivité des divers groupes hydroxyles de la cellulose, la position 6 est estérifiée la première, les positions 2 et 3 sont estérifiées ensuite. Il existe divers grades de nitrocellulose qui se différencient par :
- leur degré de polymérisation, ou encore le nombre d'unités anhydroglucose,
- le degré de substitution c'est à dire le nombre moyen de groupes nitrates par cycle anhydroglucose. Pour une nitrocellulose totalement estérifiée, le degré de substitution est de 3 ce qui correspond à 14.1 % d'azote. Pour un degré de substitution de 2, le taux d'azote est de 11.1 %.

Pour un taux d'azote donné, la nitrocellulose comprend trois types de cycles anhydroglucoses : des cycles trinitrés, des cycles dinitrés et des cycles mononitrés. Considérant la grande réactivité du groupe hydroxyle en position 6 par rapport à celle des groupes en position 2 et 3, la présence de cycles non nitrés est très faible.

Par exemple, pour un grade de nitrocellulose contenant 12.2 % d'azote, soit un degré de substitution de 2.3, le polymère comprend environ :
- 50 % de cycles trinitrés,
- 34 % de cycles dinitrés,
- 16 % de cycles mononitrés.

Le phénomène de jaunissement de solutions de nitrocellulose ou de formulations contenant de la nitrocellulose est un phénomène connu des formulateurs de vernis pour bois et de vernis cosmétiques. Ce jaunissement est dû à une décomposition quasi-spontanée de la nitrocellulose par une dénitrification des fonctions esters nitriques. Cette dénitrification a principalement lieu sur les positions 2 et 3, la position 6 (fonction ester primaire) étant plus stable à température ambiante. A des températures plus élevées, le phénomène est plus aléatoire et peut avoir lieu au niveau de toutes les fonctions esters. La température est donc un accélérateur de cette décomposition et du jaunissement (Cellulose Nitrate in Conservation, Charles Selwitz, Guetty Conservation Institute, 1988).

Les rayonnements ultraviolets sont également responsables du jaunissement de la nitrocellulose par la saponification des groupes esters comme précédemment, voire même l'ouverture des cycles anhydroglucoses. Il est important de noter qu'une intensité lumineuse de forte énergie n'est pas forcément nécessaire pour réaliser ce phénomène.

Des solutions existent pour limiter ce phénomène. Par exemple, il a été démontré que ce phénomène est accentué par la présence en nombre important de cycles trinitrés. D'autres voies ont été envisagées pour les nitrocelluloses utilisées dans les explosifs ayant un taux d'azote supérieur à 12.4 % comme l'utilisation de stabilisants comme :
- la diphénylamine,
- le méthyldiphénylurée,
- la 2-nitrodiphénylamine,
- l'urée,
- le dicyandiamide,
- l'oxamide.

Toutefois, ces composés ne peuvent être utilisés dans les milieux cosmétiques pour des raisons de dangerosité et de législation cosmétique.

D'autres filmogènes sont connus de l'homme de l'art en remplacement de la nitrocellulose dans les formulations transparentes. Ces possibilités de remplacement offrent des propriétés intéressantes par l'absence ou le faible jaunissement. On citera notamment :
- les polymères de méthacrylate d'éthyle (US 4,409,203) utilisés en présence de dérivés non nitrés de cellulose,
- l'acétate-butyrate de cellulose (US 5,512,273 et US 5,130,125),
- l'éthylcellulose.

Cependant, ces différents filmogènes n'offrent pas les mêmes propriétés d'adhérence sur l'ongle. Ils n'apportent pas non plus la même brillance et sont, de plus, beaucoup plus coûteux. La nitrocellulose reste donc un ingrédient de choix pour la formulation de vernis à ongles malgré l'inconvénient cité plus haut.

### Objet de l'invention

Un objet de la présente invention est la formulation de vernis à ongles transparents, contenant de la nitrocellulose, applicables directement sur l'ongle ou sur un vernis coloré pour en augmenter la brillance, et présentant un taux de jaunissement plus faible que les formulations antérieures.

Conformément à l'invention, cet objectif est atteint par une composition cosmétique anhydre selon la revendication 1.

### Description générale de l'invention

Afin de résoudre le problème mentionné ci-dessus, la présente invention propose une composition cosmétique anhydre pour vernis à ongles, comprenant au moins un solvant organique cosmétiquement acceptable, de la nitrocellulose et une quantité efficace d'au moins un agent de réduction du jaunissement, dans laquelle le au moins un agent de réduction du jaunissement est un dérivé triester de l'acide trimellitique choisi parmi les trimellitates d'alcools gras.

En effet, les inventeurs ont découvert d'une manière surprenante que lesdits dérivés d'acide trimellitique permettaient de réaliser l'objectif de réduire nettement le jaunissement de compositions comprenant de la nitrocellulose.

Par « quantité efficace d'au moins un agent de réduction du jaunissement », on entend une quantité suffisante pour obtenir une réduction notable et significative du jaunissement de la composition cosmétique. Cette quantité minimale en agent de réduction du jaunissement à mettre en oeuvre, qui peut varier selon la nature du solvant cosmétiquement acceptable retenu pour la composition, de la nature et de la quantité de nitrocellulose utilisée, ainsi qu'en fonction des autres ingrédients choisis, peut être déterminée sans aucune difficulté au moyen d'un test classique de mesure du jaunissement, tel que celui donné dans les exemples ci-après. En pratique, le ou les agent(s) de réduction du jaunissement représente(nt) en général 0.01 à 1 % en poids de la composition totale, de préférence 0.02 à 0.8 % en poids, en particulier de 0.05 à 0.5 % en poids de la composition totale.

Par conséquent, l'avantage de la présente invention consistant à proposer l'utilisation de dérivés triesters de l'acide trimellitique est une réduction significative du jaunissement de la formulation.

D'autre part, l'ajout des agents de réduction du jaunissement selon l'invention n'affecte pas négativement les autres qualités des compositions, à savoir les bonnes caractéristiques d'application, le temps de séchage, la durabilité dans le temps et la brillance.

Un avantage additionnel important de la présente invention est que les compositions cosmétiques anhydre, de préférence les formulations de vernis à ongles, nettement moins enclins au jaunissement peuvent comprendre les ingrédients habituellement utilisés par l'homme de l'art. L'homme de métier n'est donc pas obligé de reconsidérer tous les ingrédients et leurs effets, s'il souhaite utiliser les présents agents d'amélioration de la brillance. Les présents agents de réduction du jaunissement présentent par conséquent une très bonne compatibilité tant avec les solvants, qu'avec les ingrédients courants dans le domaine.

Les solvants utilisables dans l'invention sont choisis par exemple parmi les suivants : l'acétate de butyle, l'acétate d'isobutyle, l'acétate de propyle, l'acétate de méthyle, l'acétate d'éthyle, le toluène, l'isopropanol, le diacétone alcool, l'éthanol dénaturé (avec la méthyléthylcétone ou le diéthyléther), le n-butanol, les hydrocarbures linéaires ou cycliques, comme l'hexane, le cyclohexane, l'heptane, l'isododécane. On citera également les dérivés de paraffine de type Isopar qui sont des dérivés paraffiniques qui ont des chaînes carbonées de taille variable, ainsi que les glycols comme l'éthylène glycol, le propylène glycol, le dipropylène glycol ou le tripropylène glycol.

Les solvants ou leurs mélanges peuvent être utilisés entre 5 et 80 % en poids de la formulation totale. Ils sont sélectionnés selon leurs propriétés physico-chimiques (température d'ébullition par exemple) et leur aptitude à solubiliser les autres constituants de la formulation. Ils seront également sélectionnés selon leurs propriétés organoleptiques (odeur principalement) et leurs possibles utilisations selon les réglementations en vigueur.

Les compositions selon l'invention sont anhydres, c'est-à-dire elles ne contiennent a priori pas d'eau. Il est néanmoins clair que de l'eau peut se trouver en de faibles quantités dans les compositions finales, notamment introduites via l'incorporation de certains ingrédients. Par conséquent, le terme « anhydre » dans le présent contexte est à interpréter comme signifiant une teneur en eau libre inférieure à 5 % en poids, de préférence inférieure à 1 % en poids et en particulier inférieure à 0.5 % en poids de la compositions totale.

Dans les agents de réduction du jaunissement selon l'invention, les alcools gras du dérivé de l'acide trimellitique sont identiques ou différents et sont choisis indépendamment parmi les alcools gras saturés ou insaturés, linéaires ou ramifiés, en C₆-C₃₀. Par conséquent, les résidus d'alcool gras peuvent présenter une ou plusieurs insaturations et/ou comporter des ramifications. Ils comprennent d'une manière générale de 6 à 30 atomes de carbone, de préférence de 7 à 25 atomes de carbone et en particulier de 8 à 20 atomes de carbone.

Des exemples particulièrement préférés de dérivés trimellitate d'alcools gras identiques ou différents sont choisis parmi le trimellitate de triéthylhexyle, le trimellitate de tridécyle, le trimellitate de triisodécyle, le trimellitate mixte de tri(caprylyle/capryle).

De préférence, ces compositions comprenant en outre un ou plusieurs autres additifs choisis parmi les agents et co-agents filmogènes autres que la nitrocellulose, les résines, les plastifiants, les absorbants UV, les modificateurs de surface, les agents de coloration et les agents dits « traitants ».

Les agents filmogènes sont sélectionnés pour leur aptitude à former un film après séchage (évaporation partielle ou totale du ou des solvants). Il s'agit d'une part de la nitrocellulose qui peut être classée de la manière suivante :
- Nitrocellulose ayant un taux d'azote compris entre 11.8 et 12.3 % (ce type de nitrocellulose porte généralement le nom de grade RS, il s'agit d'un grade de nitrocellulose soluble dans les solvants de type ester. A l'intérieur de ce grade de type RS, il est possible de trouver des sous-grades qui se différencient notamment par leur poids moléculaire et, par voie de conséquence par la viscosité qu'ils apportent lors de leur mise en solution. Les grades utilisables dans l'invention pourront aller du grade RS 30-35 cps au grade RS 60-80 sec. La nomenclature européenne mentionne des grades de nitrocellulose allant de E24 au grade E130.
- Nitrocellulose ayant un taux d'azote inférieur 11.8 % (ce type de grade porte généralement le nom de grade SS), il s'agit d'un grade de nitrocellulose soluble dans les solvants de type alcool. Le taux d'azote est compris entre 10.7 % et 11.3 %. Comme pour le grade RS, les nitrocelluloses de grade SS peuvent se différencier par leur poids moléculaire et par voie de conséquence par la viscosité de la solution de nitrocellulose dans un solvant approprié.

La nitrocellulose se présente sous la forme d'une poudre blanche humide. La nitrocellulose est en effet commercialisée sous forme « mouillée » (ou « damped ») avec plusieurs types de solvant comme l'éthanol, l'isopropanol ou encore l'eau.

D'autre part, la composition cosmétique selon l'invention peut comprendre d'autres agents ou co-agents filmogènes. Parmi ces co-filmogènes envisageables, on citera :
- L'acétate-butyrate de cellulose. Il existe de nombreux grades de cet agent filmogène qui se différencient par le taux d'estérification de la cellulose mais aussi par le rapport du nombre de fonction ester acétate et du nombre de fonction ester butyrate. Comme pour la nitrocellulose, le cellulose acétate butyrate peut également se différencier par la longueur du polymère cellulose utilisé pour produire le filmogène.
- Ethylcellulose de différents grades de viscosité.
- Hydroxypropylcellulose de différents grades de viscosité.

Les agents et co-agents filmogènes sont utilisés dans la formule, seuls ou sous la forme de mélanges dans des proportions allant de 1 % à 17 % en poids de la composition totale, y compris la nitrocellulose.

Les résines permettent de donner du collant et de l'adhérence au film sur l'ongle. Les résines pouvant être utilisées dans l'invention sont les suivantes :
- Résine tosylamide/formaldéhyde (résine toluènesulfonamide/ formaldéhyde) vendue sous la dénomination commerciale Ketjenflex MH, Ketjenflex MS-80 par Akzo Nobel ou encore Sulfonex par Estron.
- Résine tosylamide/époxy (résine toluènesulfonamide/époxy) vendue sous la dénomination commerciale de Lustrabrite S ou Lustrabrite S-70 ou Nagellite 3050 par Telechemische ou encore sous la dénomination polytex resin par Estron.
- Copolymère acide adipique/néopentyl glycol/anhydride trimellitique vendue sous la dénomination commerciale Uniplex 670-P par Unitex ou encore Chempol 509-9514 par Cook Composites
- Copolymère d'anhydride phtalique/glycérine/glycidyl décanoate
- Copolymère d'anhydride phtalique/anhydride trimellitique/glycols vendue sous la dénomination commerciale Polynex Resin par Estron ou encore 7809 Polynex Resin par Degen
- Copolymère de glycérine/acide phtalique
- Copolymère styrène/acrylates/acrylonitrile
- Polymères et copolymères d'acrylates
- Copolymères d'acrylates et de styrène
- Sucrose acétate isobutyrate vendue sous la dénomination commerciale SAIB par Eastman
- Résine polyvinylbutyral.

En plus de leur action collante, la plupart de ces résines ont également une action plastifiante. Les résines sont utilisées dans la formule seules ou sous la forme de mélanges dans des quantités allant de 0.2 à 15 % en poids total de la formulation.

Les plastifiants sont des composés ayant une température d'ébullition élevée qui ne s'évaporent pas lors du séchage du vernis à ongles. Ils sont utilisés pour moduler la dureté du film formé afin d'obtenir les caractéristiques physico-chimiques du film souhaitées.

Des exemples de plastifiants utilisables dans l'invention sont l'acétyl tributyl citrate, l'acétyl triéthyl citrate, le tributyl citrate, le triéthylcitrate, le dibutyl phtalate, le triphénylphosphate, la triacétine, le triméthyl pentanyl diisobutyrate, la triéthylhexanoïne, le sucrose benzoate, le dibutyl adipate, le diéthyl phthalate, le diisobutyl adipate, le diisopropyl adipate, le dipropylène glycol dibenzoate, etc.

Parmi les absorbants UV, on citera les absorbants de lumière UV qui permettent de protéger la formulation ou les agents colorants utilisés l'invention des rayonnements UV. Parmi les protecteurs UV utilisables dans l'invention, on citera : la benzophénone-1 (CAS 131-56-6), la benzophénone-3 (CAS 131-57-7), le benzyl salicylate (CAS 118-58-1), l'étocrylene (CAS 5232-99-5), le drométrizole (CAS 2440-22-4), le butyl méthoxydibenzoylméthane (CAS 70356-09-1), etc.

Les absorbants UV seront utilisés seuls ou sous la forme de combinaison dont le pourcentage total dans la formule peut aller en général de 0.1 à 1 % en poids.

Parmi les additifs utilisables selon l'invention, on citera également les modificateurs de surface comme les cyclométhicones ou cyclopentasiloxane, les diméthicones et le triméthylsiloxysilicate.

Ces additifs de surface sont généralement utilisés dans des faibles quantités dans la formulation allant de 0.001 à 0.5 % en poids total de la formulation.

On citera également les additifs dits « traitants » de l'ongle utilisable dans la composition selon l'invention. Parmi ces additifs « traitants », on citera :
- le formaldéhyde utilisé comme durcisseur de l'ongle,
- la vitamine E acétate (tocophéryl acétate),
- la vitamine A palmitate (rétinyl palmitate),
- les dérivés organiques ou inorganiques de calcium tels que le chlorure de calcium, le pantothénate de calcium,
- le diméthyloxobenzodioxasilane vendu sous la dénomination commerciale Pro-DSB par Exsymol,
- la myrrhe,
- les acides aminés soufrés comme la cystéine, ses sels et leurs dérivés, la cystine, le glutathion. Les acides aminés soufrés sont en effet capables de créer des cross-linkings entre la kératine et les groupes SH libres de ces dérivés soufrés,
- la kératine hydrolysée d'origine végétale comme le Prosina de Croda,
- les alpha-hydroxyacides comme l'acide citrique, l'acide ascorbique,
- la biotine,
- l'urée et la diméthylurée.

La composition selon l'invention pourra comprendre des matières colorantes comme les pigments, les nacres ou les glitters.

Parmi les pigments utilisables dans l'invention on citera le dioxyde de titane (CI 77891), l'oxyde de fer noir (CI 77499), l'oxyde de fer rouge (CI 77491 ), le DC Red 6 Ba Lake (CI 15850), le DC Red 7 Ca Lake (CI 15850:1), le DC Red 34 Ca Lake (CI 15880), le FDC Yellow 5 Al Lake (CI 19140), le FDC Blue 1 Al Lake (CI 42090), le bleu ultramarine (CI 77007), le DC Violet 2 (CI 60725), le DC Black 2 (CI 77266), etc.

Les nacres sont également utilisables dans l'invention, les nacres sont des particules minérales de différentes natures recouvertes d'une ou plusieurs couches d'oxydes (oxydes de titane ou oxydes de fer) ou encore de pigments. Les nacres peuvent se présenter sous la forme d'une poudre ou de plaque de tailles variées. Parmi les nacres utilisables dans l'invention, on peut citer :
- les nacres préparées à partir de mica naturel et ayant subi des opérations de coating successives,
- les nacres préparées à partir de mica synthétique appelé également synthetic fluorphlogopite,
- les nacres préparées à partir de calcium sodium borosilicate,
- les nacres préparées à partir d'oxyde de silice.

Les glitters utilisables dans l'invention peuvent être préparés à partir de plusieurs types de matériaux comme les films polyesters comportant en surface une couche métallique, le polyéthylène téréphtalate, le polybutylène téréphtalate, les copolymères d'acrylates, les copolymères d'acrylates ou encore l'aluminium.

Dans un aspect supplémentaire, l'invention propose donc l'utilisation d'un ou de plusieurs trimellitates d'alcools gras dans la préparation d'une composition cosmétique anhydre comme agents de réduction du jaunissement.

L'invention concerne également un procédé de préparation d'une composition cosmétique anhydre, notamment pour vernis à ongles ou de soins des ongles, dans lequel on mélange au moins un solvant organique cosmétiquement acceptable, de nitrocellulose, au moins un agent de réduction du jaunissement choisi parmi les trimellitates d'alcools gras, ainsi qu'éventuellement un ou de plusieurs autres additifs choisis parmi les agents et co-agents filmogènes autres que la nitrocellulose, les résines, les plastifiants, les absorbants UV, les modificateurs de surface, les agents de coloration et les agents dits « traitants ».

Il est à noter que les agents de réduction du jaunissement peuvent non seulement être ajoutés à n'importe quel moment du procédé, c'est-à-dire que l'effet des présents agents n'est pas affecté par l'ordre dans lequel les ingrédients sont dosés.

De plus, en complément des avantages déjà mentionnés ci-dessus en relation avec les compositions, il est à remarquer que le procédé ci-dessus peut être réalisé moyennant des installations tout à fait standard et ne demande pas de connaissance, ni de précautions particulières de l'opérateur.

En conclusion, les inventeurs ont découvert d'une manière surprenante que lesdits dérivés d'acide trimellitique permettaient de réaliser l'objectif de réduire nettement le jaunissement de compositions comprenant de la nitrocellulose.

### Exemples

Parmi les dérivés d'acide trimellitique préférés, on peut citer :
- le Tridécyl Trimellitate (CAS 94109-09-8) vendu sous la dénomination Liponate TDTM par Lipo, Pelemol TDTM par Phoenix ou encore Dub TMTD par Stéarinerie Dubois.
- Le Triisodécyl Trimellitate (CAS 36631-30-8) vendu sous la dénomination Dub TMI par Stéarinerie Dubois.
- Le Triéthylhexyl Trimellitate (CAS 3319-31-1) vendu sous la dénomination Bisoflex TOT par Cognis Performance Chemicals ou EastmanTOTM par Eastman Chemical, ou encore Dub TMO par Stéarinerie Dubois.
- Tricaprylyl/capryl trimellitate (CAS 90218-76-1) vendu sous la dénomination commerciale Biosynth TM 810 par la société Biosynthis.

Ci-dessous dans le Tableau 1, quelques exemples de formulations selon l'invention illustrent la diminution du jaunissement. La formulation de l'Ex 1 étant donné à titre comparatif et représente une composition connue de l'état de la technique.

Les formulations sont réalisées selon les modes opératoires connus de l'homme de l'art. Il s'agit de formulations transparentes et limpides.

Les échantillons ont subi un vieillissement accéléré (température et lumière de forte intensité) par l'utilisation d'une chambre de test au xénon (appareil Q-Sun 1000) pendant 72 heures.

Cette méthode pour accélérer le jaunissement comprend les étapes suivantes : les formulations sont conditionnées dans les flacons de vernis à ongles utilisés habituellement dans le commerce d'une contenance de 10 ml. Les flacons remplis sont ensuite placés dans la chambre de test d'un appareil de test de lumière accéléré (appareil Q-Sun 1000 vendu par la société Labomat) pendant 72 heures. Cet appareil combine deux effets : celui de la lumière de forte intensité et celui de la température.

Le jaunissement est mesuré par spectrophotomètre visible (échelle Gardner) et la couleur de la formulation est évaluée par mesure par un colorimètre Lico 50 de la société Dr Lange dans des cuvettes de 10 mm de diamètre.

Tableau 1

| **Ingrédients** | **Ex 1** | **Ex 2** | **Ex 3** | **Ex 4** | **Ex 5** |
|---|---|---|---|---|---|
| Acétate d'éthvle | 50 | 50 | 50 | 50 | 50 |
| Acétate de butyle | 16.7 | 16.6 | 16.6 | 16.6 | 16.6 |
| Nitrocellulose (70 %) | 15 | 15 | 15 | 15 | 15 |
| Résine polyester | 9 | 9 | 9 | 9 | 9 |
| Copolymère styrène/acrylique | 3 | 3 | 3 | 3 | 3 |
| Acétyl tributyl citrate | 6 | 6 | 6 | 6 | 6 |
| Etocrylene | 0.3 | 0.3 | 0.3 | 0.3 | 0.3 |
| Tridécyl Trimellitate | | 0.1 | | | |
| Triisodécyl Trimellitate | | | 0.1 | | |
| Triéthylhexyl Trimellitate | | | | 0.1 | |
| Tricaprylyl/capryl Trimellitate | | | | | 0.1 |
| Total | 100 | 100 | 100 | 100 | 100 |
| Jaunissement | 352 | 67 | 75 | 62 | 79 |

On constate que le jaunissement est réduit de façon significative dans les Exemples 2 à 5 par comparaison à l'Exemple 1 non conforme à l'invention.

## Revendications

1. Composition cosmétique anhydre pour vernis à ongles, comprenant au moins un solvant organique cosmétiquement acceptable, de la nitrocellulose et une quantité efficace d'au moins un agent de réduction du jaunissement, **caractérisée en ce** le au moins un agent de réduction du jaunissement est un dérivé triester de l'acide trimellitique choisi parmi les trimellitates d'alcools gras.

2. Composition selon la revendication 1, dans laquelle les alcools gras du dérivé de l'acide trimellitique sont identiques ou différents et sont choisis indépendamment parmi les alcools gras saturés ou insaturés, linéaires ou ramifiés, en C₆-C₃₀.

3. Composition selon la revendication 2, dans laquelle les alcools gras sont choisis parmi le trimellitate de triéthylhexyle, le trimellitate de tridécyle, le trimellitate de triisodécyle, le trimellitate de tri(caprylyle/capryle).

4. Composition selon l'une quelconque des revendications précédentes, dans laquelle le au moins un agent de réduction du jaunissement représente 0.01 à 1 % en poids de la composition totale.

5. Composition selon l'une quelconque des revendications précédentes, comprenant en outre un ou plusieurs autres additifs choisis parmi les agents et co-agents filmogènes autres que la nitrocellulose, les résines, les plastifiants, les absorbants UV, les modificateurs de surface, les agents de coloration et les agents dits « traitants ».

6. Utilisation d'un ou de plusieurs trimellitates d'alcools gras dans la préparation d'une composition cosmétique anhydre comme agents de réduction du jaunissement.

7. Procédé de préparation d'une composition cosmétique anhydre selon l'une quelconque des revendications 1 à 5, comprenant le mélange d'au moins un solvant organique cosmétiquement acceptable, de nitrocellulose, d'au moins un agent de réduction du jaunissement choisi parmi les trimellitates d'alcools gras, ainsi qu'éventuellement d'un ou de plusieurs autres additifs choisis parmi les agents et co-agents filmogènes autres que la nitrocellulose, les résines, les plastifiants, les absorbants UV, les modificateurs de surface, les agents de coloration et les agents dits « traitants ».
